(19) Europäisches Patentamt
European Patent Office
Office européen des brevets

(11) Veröffentlichungsnummer: **0 229 301**
**A1**

(12)
# EUROPÄISCHE PATENTANMELDUNG

(21) Anmeldenummer: 86117015.7

(22) Anmeldetag: 08.12.86

(51) Int. Cl.[3]: **C 07 D 249/08**
A 01 N 43/653, C 07 D 303/48
C 07 B 53/00
//C07C131/00

(30) Priorität: 19.12.85 DE 3545034

(43) Veröffentlichungstag der Anmeldung:
22.07.87 Patentblatt 87/30

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Kranz, Eckart, Dr.
Am Acker 9
D-5600 Wuppertal 1(DE)

(72) Erfinder: Stetter, Jörg, Dr.
Gellertweg 4
D-5600 Wuppertal 1(DE)

(72) Erfinder: Brandes, Wilhelm, Dr.
Eichendorffstrasse 3
D-5653 Leichlingen(DE)

(72) Erfinder: Reinecke, Paul, Dr.
Steinstrasse 8
D-5090 Leverkusen 3(DE)

(54) **Diastereomeres (A) des 3,3-Dimethyl-1(4-methoximino-methyl-phenoxy)-1-(1,2,4-triazol-1-yl)-butan-2-ols.**

(57) Das neue Diastereomere (A) des 3,3-Dimethyl-1-(4-methoximinomethyl-phenoxy)-1-(1,2,4-triazol-1-yl)-butan-2-ols der Formel I

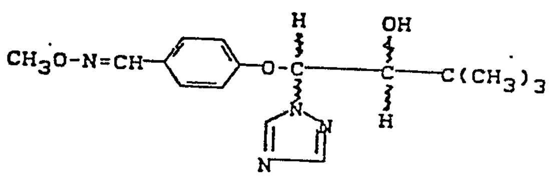

(I)

ein Verfahren zur Herstellung des neuen Wirkstoffes und dessen Verwendung als Fungizid.

Das neue 3,3-Dimethyl-1-(4-methoximino-methyl-phenoxy)-trans-1,2-epoxy-butan der Formel IV

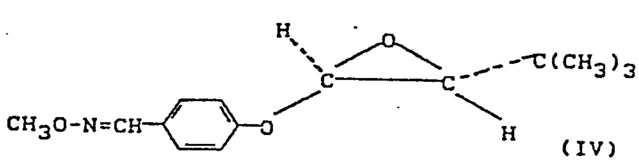

(IV)

EP 0 229 301 A1

BAYER AKTIENGESELLSCHAFT
Konzernverwaltung RP
Patentabteilung

5090 Leverkusen, Bayerwerk

Dü/ABc
IV b

Diastereomeres (A) des 3,3-Dimethyl-1-(4-methoximino-methyl-phenoxy)-1-(1,2,4-triazol-1-yl)-butan-2-ols

Die vorliegende Erfindung betrifft das neue Diastereomere (A) des 3,3-Dimethyl-1-(4-methoximinomethyl-phenoxy)-1-(1,2,4-triazol-1-yl)-butan-2-ols, ein neues Verfahren zur Herstellung dieses Diastereomeren (A) und dessen Verwendung als Fungizid.

Es ist bereits bekannt, daß das Diastereomerengemisch des 3,3-Dimethyl-1-(4-methoximinomethyl-phenoxy)-1-(1,2,4-triazol-1-yl)-butan-2-ols fungizide Eigenschaften besitzt (vgl. EP-OS 0 076 370). Die Wirkung dieses Produktes ist gut; jedoch ist der bei sehr niedrigen Aufwandmengen erzielte Effekt nicht immer befriedigend.

Weiterhin ist bekannt, daß sich Diastereomeren-Gemische des 3,3-Dimethyl-1-(4-methoximinomethyl-phenoxy)-1-(1,2,4-triazol-1-yl)-butan-2-ols nach mehreren Verfahren herstellen lassen (vgl. EP-OS 0 076 370). So erhält man 3,3-Dimethyl-1-(4-methoximinomethyl-phenoxy)-1-(1,2,4-triazol-1-yl)-butan-2-ol in Form eines Diastereomeren-Gemisches, wenn man 3,3-Dimethyl-1-brom-1-(4-methoximinomethyl-phenoxy)-butan-2-on mit 1,2,4-Triazol umsetzt und das dabei entstehende 3,3-Dimethyl-1-(4-methoximinomethyl-phenoxy)-1-(1,2,4-triazol-1-yl)-butan-2-on mit Natriumborhydrid reduziert. Nachteilig an diesem Verfahren ist aber, daß jeweils nur Diastereomeren-Gemische anfallen, die unterschiedliche Mengen an den beiden Diastereomeren (A) und (B) aufweisen.

Es wurde nun das neue Diastereomere (A) des 3,3-Dimethyl-1-(4-methoximinomethyl-phenoxy)-1-(1,2,4-triazol-1-yl)-butan-2-ols der Formel

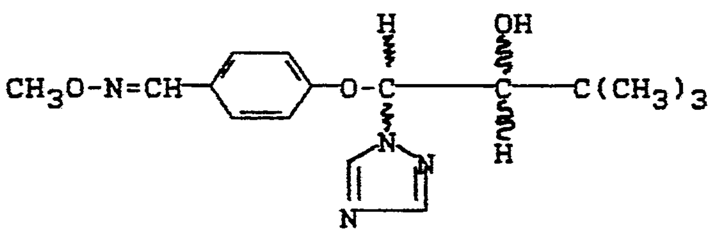

(I)

gefunden.

Weiterhin wurde gefunden, daß man das neue Diastereomere (A) des 3,3-Dimethyl-1-(4-methoximinomethyl-phenoxy)-1-(1,2,4-triazol-1-yl)-butan-2-ols der Formel (I) nach einem neuen Verfahren erhält, wenn man 1-Brom-3,3-dimethyl-1,2-epoxy-butan der Formel

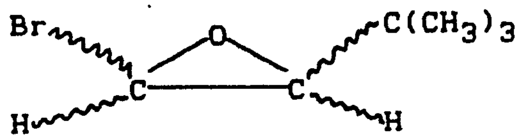

(II)

mit 4-Hydroxy-benzaldehyd-O-methyloximether der Formel

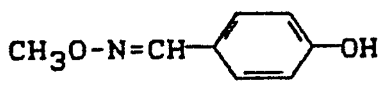

(III)

gegebenenfalls in Gegenwart einer Base sowie gegebenenfalls in Gegenwart eines Verdünnungsmittels umsetzt und das dabei anfallende 3,3-Dimethyl-1-(4-methoximinomethyl-phenoxy)-trans-1,2-epoxy-butan der Formel

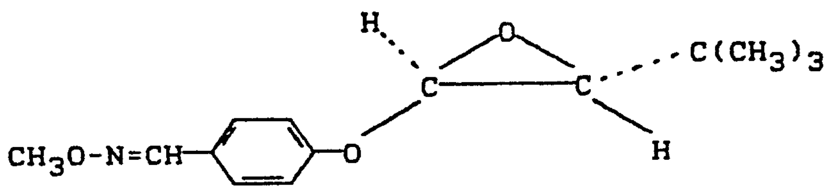

(IV)

in einem zweiten Reaktionsschritt mit 1,2,4-Triazol oder einem Salz des 1,2,4-Triazols gegebenenfalls in Gegenwart einer Base und gegebenenfalls in Gegenwart eines Verdünnungsmittels umsetzt.

Schließlich wurde gefunden, daß sich das neue Diastereomere (A) des 3,3-Dimethyl-1-(4-methoximinomethyl-phenoxy)-1-(1,2,4-triazol-1-yl)-butan-2-ols der Formel (I) durch eine sehr gute fungizide Wirksamkeit auszeichnet.

Überraschenderweise besitzt das neue Diastereomere (A) des 3,3-Dimethyl-1-(4-methoximinomethyl-phenoxy)-1-(1,2,4-triazol-1-yl)-butan-2-ols der Formel (I) wesentlich bessere fungizide Eigenschaften als das entsprechende Diastereomeren-Gemisch, das aus dem Stand der Technik als hoch wirksames Fungizid bekannt ist. Außerdem übertrifft der erfindungsgemäße Wirkstoff auch das Diastereomere (B) des 3,3-Dimethyl-1-(4-methoximinomethyl-phenoxy)-1-(1,2,4-triazol-1-yl)-butan-2-ols bezüglich der fungiziden Wirksamkeit.

Überraschenderweise ist auch, daß sich bei der Durchführung des erfindungsgemäßen Verfahrens nur das Diastereomere (A) des 3,3-Dimethyl-1-(4-methoximinomethyl-phenoxy)-1-(1,2,4-triazol-1-yl)-butan-2-ols der Formel (I) bildet, während bei den entsprechenden vorbekannten Verfahren jeweils nur Diasteromeren-Gemische anfallen.

Das erfindungsgemäße Verfahren zeichnet sich durch eine Reihe von Vorteilen aus. So sind die benötigten Ausgangsstoffe in einfacher Weise und auch in größeren Mengen zugänglich. Ferner bereiten auch die Durchführung der Umsetzung und die Isolierung des gewünschten Produktes keinerlei Schwierigkeiten. Besonders günstig ist es, daß nach dem erfindungsgemäßen Verfahren selbst dann das Diastereomere (A) des 3,3-Dimethyl-1-(4-methoximinomethyl-phenoxy)-1-(1,2,4-triazol-1-yl)-butan-2-ols der Formel (I) erhalten wird, wenn cis-trans-Gemische des 1-Brom-3,3-dimethyl-1,2-epoxy-butans der Formel (II) als Ausgangsmaterialien eingesetzt werden.

Der erfindungsgemäße Wirkstoff ist durch die Formel (I) charakterisiert. Es handelt sich hierbei um das Diastereomere (A), also um die threo- oder erythro-Form dieses Stoffes. Das erfindungsgemäße Diasteromere (A) des 3,3-Dimethyl-1-(4-methoximinomethyl-phenoxy)-1-(1,2,4-triazol-1-yl)-butan-2-ols der Formel (I) liegt als Racemat von optischen Isomeren vor.

Verwendet man ein cis-trans-Gemisch des 1-Brom-3,3-dimethyl-1,2-epoxy-butans und 4-Hydroxy-benzaldehyd-O-methyl-oximether als Ausgangsstoffe und das Natrium-Salz des 1,2,4-Triazols als Reaktionskomponente in der zweiten Stufe der Umsetzung, so kann der Verlauf des erfindungsgemäßen Verfahrens durch das folgende Formelschema veranschaulicht werden:

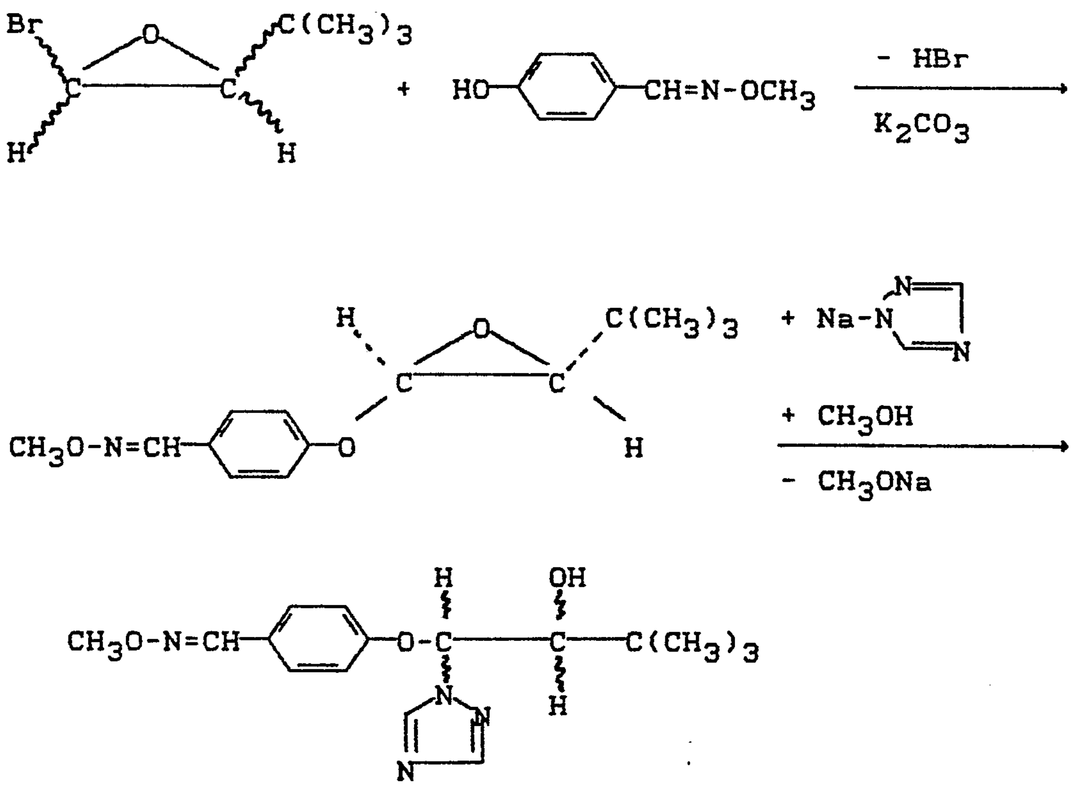

Das bei dem erfindungsgemäßen Verfahren als Ausgangsstoff benötigte 1-Brom-3,3-dimethyl-1,2-epoxy-butan der Formel (II) kann als cis-trans-Gemisch oder auch in Form des trans-Isomeren eingesetzt werden. Man erhält 1-Brom-3,3-dimethyl-1,2-epoxy-butan, indem man Pinakolon durch Bromierung in das α, α-Dibrom-keton der Formel

$$Br_2CH\text{-}CO\text{-}C(CH_3)_3 \qquad (V)$$

überführt, welches dann bei der anschließenden Reduktion unter alkalischen Bedingungen cyclisiert wird.

Der bei dem erfindungsgemäßen Verfahren außerdem als Ausgangsstoff benötigte 4-Hydroxybenzaldehyd-O-methyl-oximether der Formel (III) ist bekannt (vgl. EP-OS 0 76 370).

Das bei der Durchführung des erfindungsgemäßen Verfahrens als Zwischenprodukt auftretende 3,3-Dimethyl-1-(4-methoximino-methyl-phenoxy)-trans-1,2-epoxy-butan der Formel (IV) ist bisher noch nicht beschrieben worden.

Bei der Durchführung des erfindungsgemäßen Verfahrens wird der 4-Hydroxy-benzaldehyd-O-methyl-oximether entweder in Gegenwart von Basen oder aber in Form eines Salzes eingesetzt. Als Basen kommen hierbei vorzugsweise Alkalimetallcarbonate, wie Natrium- oder Kaliumcarbonat, ferner Alkalimetallhydroxide, wie Natrium- oder Kaliumhydroxid, außerdem Hydride, wie Natriumhydrid, und weiterhin Alkoholate, wie Kalium-tert.-butylat, in Betracht. Als Salze von der Verbindung der Formel (III) kommen vorzugsweise das Natrium- oder das Kalium-Salz in Frage.

Als Verdünnungsmittel können bei der Durchführung des erfindungsgemäßen Verfahrens in der ersten Stufe alle für derartige Reaktionen üblichen inerten, organischen Solventien eingesetzt werden. Vorzugsweise in Betracht kommen Ketone, wie Aceton, außerdem Nitrile, wie Acetonitril, ferner aromatische Kohlenwasserstoffe, wie Benzol, Toluol und Xylol, weiterhin Ether, wie Diethylether und Tetrahydrofuran, und außerdem polare, aprotische Lösungsmittel, wie Dimethylformamid und Dimethylsulfoxid.

Bei der Durchführung des erfindungsgemäßen Verfahrens wird in der zweiten Stufe das 1,2,4-Triazol entweder in Gegenwart von Basen, wie Kalium- oder Natriumhydroxid, oder aber in Form des Triazol-Natrium- oder Triazol-Kalium-Salzes eingesetzt.

Als Verdünnungsmittel kommen bei der Durchführung der zweiten Stufe des erfindungsgemäßen Verfahrens vorzugsweise Alkohole, wie Methanol, Ethanol oder n-Butanol, in Frage.

Die Reaktionstemperaturen können bei der Durchführung des erfindungsgemäßen Verfahrens innerhalb eines bestimmten Bereiches variiert werden. Bei der Durchführung der ersten Stufe arbeitet man im allgemeinen bei Temperaturen zwischen -10°C und +100°C, vorzugsweise zwischen 0°C und 50°C. Bei der Durchführung der zweiten Stufe arbeitet man im allgemeinen bei Temperaturen zwischen 50 und 120°C, vorzugsweise zwischen 60 und 100°C.

Das erfindungsgemäße Verfahren wird im allgemeinen unter Normaldruck durchgeführt. Es ist jedoch auch möglich, unter erhöhtem oder vermindertem Druck zu arbeiten.

Bei der Durchführung des erfindungsgemäßen Verfahrens setzt man die Ausgangsstoffe und die Reaktionskomponenten im allgemeinen in äquimolaren Mengen ein. Es ist aber auch möglich, die eine oder andere Komponente in einem Überschuß zu verwenden. Die Aufarbeitung erfolgt nach üblichen Methoden.

Geht man bei der Durchführung des erfindungsgemäßen Verfahrens so vor, daß man 1-Brom-3,3-dimethyl-1,2-epoxy-butan der Formel (II) zunächst mit 1,2,4-Triazol in Gegenwart einer Base oder mit einem Salz des 1,2,4-Triazols umsetzt und das dabei entstehende 3,3-Dimethyl-1-(1,2,4-triazol-1-yl)-trans-1,2-epoxy-butan der Formel

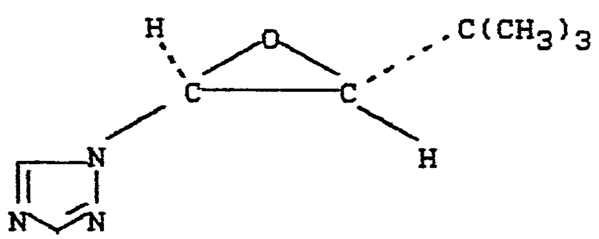

(VI)

dann im zweiten Reaktionsschritt mit 4-Hydroxy-benzaldehyd-O-methyl-oximether der Formel (III) gegebenenfalls in Gegenwart einer Base umsetzt, so erhält man das Diastereomere (B) des 3,3-Dimethyl-1-(4-methoximinomethyl-phenoxy)-1-(1,2,4-triazol-1-yl)-butan-2-ols.

Im übrigen läßt sich das Diastereomere (B) der Verbindung der Formel (I) auch aus dem bekannten Diastereomeren-Gemisch nach üblichen Methoden isolieren.

Der erfindungsgemäße Wirkstoff weist eine starke mikrobizide Wirkung auf und kann zur Bekämpfung von unerwünschten Mikroorganismen praktisch eingesetzt werden. Der Wirkstoff ist für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans,
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;

Sphaerotheca-Arten, wie Sphaerotheca fuliginea;
Podosphaera-Arten, wie Venturia inaequalis;
Pyrenophora-Arten, wie Pyrenophora teres oder P. graminea;
(Konidienform; Drechslera, Syn: Helminthosporium);
Cochliobolus-Arten, wie Cochliobocus sativus;
(Konidienform; Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit des erfindungsgemäßen Wirkstoffs in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel kann der erfindungsgemäße Wirkstoff mit besonders gutem Erfolg zur Bekämpfung von

Sphaerotheca-Arten, wie Sphaerotheca fuliginea, an der Gurke, und zur Bekämpfung von Fusarium-Arten, wie Fusarium culmorum, an Weizen eingesetzt werden.

Der erfindungsgemäße Wirkstoff kann in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Die Formulierungen werden in bekannter Weise hergestellt, z.B. durch Vermischen des Wirkstoffs mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid

und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und systhetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive könne mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgmeäße Wirkstoff kann in den Formulierungen in Mischung mit anderen bekannten Wirkstoffen vorliegen wie Fungizide, Insektizide, Akarizide und Herbizide sowie Mischungen mit Düngemitteln und anderen Wachstumsgulatoren.

Der Wirkstoff kann als solcher, in Form seiner Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, den Wirkstoff nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz des erfindungsgemäßen Stoffs als Fungizid kann die Aufwandmenge je nach Art der Applikationen in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,02 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellung und Verwendung des erfindungsgemäßen Wirkstoffs gehen aus den folgenden Beispielen hervor.

## Herstellungsbeispiele

### Beispiel 1

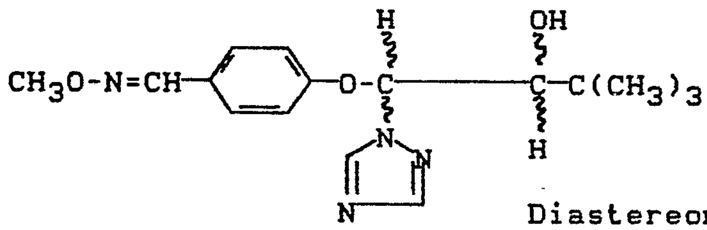

Diastereomeres (A)

3,2 g (0,06 Mol) Natriummethylat werden in 40 ml trockenem Methanol gelöst und mit 4,1 g (0,06 Mol) 1,2,4-Triazol versetzt. Zu dieser Lösung tropft man unter Rühren bei Rückflußtemperatur eine Lösung von 5,0 g (0,02 Mol) 3,3-Dimethyl-1-(4-methoxyimino-methyl-phenoxy)-trans-1,2-epoxy-butan in 10 ml trockenem Methanol. Das Reaktionsgemisch wird 20 Stunden unter Rückfluß nachgerührt und dann eingeengt. Der verbleibende Rückstand wird in Wasser/Essigester aufgenommen. Die Phasen werden getrennt, und die wäßrige Phase wird dreimal mit Essigester extrahiert. Die vereinigten organischen Phasen werden nacheinander je dreimal mit 3 N wäßriger Natronlauge und Wasser gewaschen. Nach dem Trocknen der organischen Phase über Natriumsulfat und Abziehen des Lösungsmittels unter vermindertem Druck erhält man 4,5 g eines Rohproduktes, das durch Chromatographie über eine Kieselgel-Säule mit dem Fließmittel Methylenchlorid/Methanol = 19:1 gereinigt wird. Nach dem Einengen des Eluates erhält man 1,92 g (30 % der Theorie) an dem Diastereomeren (A) des 3,3-Dimethyl-1-(4-methoximinomethyl-phenoxy)-1-(1,2,4-triazol-1-yl)-butan-2-ols in Form eines zähflüssigen Öles.

Herstellung des 3,3-Dimethyl-1-(4-methoximinomethyl-phenoxy)-trans-1,2-epoxy-butans:

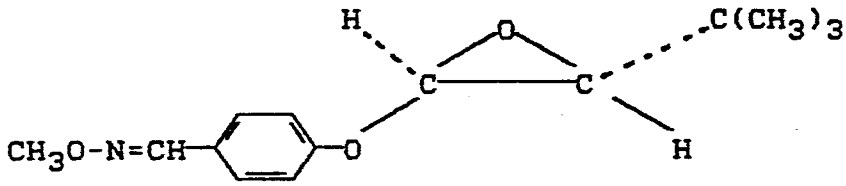

Zu einer Mischung aus 15,1 g (0,1 Mol) 4-Hydroxy-benzaldehyd-O-methyl-oximether und 15,4 g (0,11 Mol) fein gepulvertem Kaliumcarbonat in 250 ml Aceton werden unter Rühren 17,9 g (0,1 Mol) cis/trans (10/90) 1-Brom-3,3-dimethyl-1,2-epoxybutan getropft. Nach Beendigung der leicht exothermen Reaktion wird noch 3 Stunden unter Rückfluß nachgerührt. Nach dem Abfiltrieren des Niederschlages wird die Lösung unter vermindertem Druck eingeengt. Man erhält 18,6 g (75 % der Theorie) an 3,3-Dimethyl-1-(4-methoxyiminomethyl-phenoxy)-trans-1,2-epoxybutan.

Herstellung von 1-Brom-3,3-dimethyl-1,2-epoxy-butan:

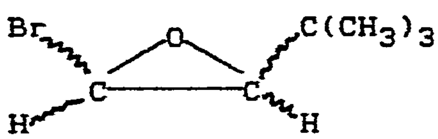

Eine Lösung von 51,6 g (0,2 Mol) 1,1-Dibrom-3,3-dimethylbutanon-2 in 80 ml Diethylether wird in eine Lösung von 14 g (0,35 Mol) Natriumhydroxid in 60 ml Wasser und 25 ml Methanol getropft. Unter Kühlung auf -10° C werden unter Rühren portionsweise 5 g (0,132 Mol) Natriumborhydrid eingetragen. Nach Abklingen der Reaktion wird noch weitere

24 Stunden bei Raumtemperatur nachgerührt. Die organische Phase wird abgetrennt, die wäßrige Phase zweimal mit Ether ausgeschüttelt, die vereinigten organischen Phasen werden getrocknet und bei 30° C im Vakuum über eine 1 m lange Vigreuxkolonne vorsichtig eingeengt und anschließend destilliert.

Ausbeute: 22,8 g (63,7 % der Theorie) an 1-Brom-3,3-dimethyl-1,2-epoxybutan als 1:9-Gemisch der cis/trans-Isomere.

Herstellung von 1,1-Dibrom-3,3-dimethyl-butan-2-on:

$$Br_2CH\text{-}CO\text{-}C(CH_3)_3$$

In eine Mischung von 100 g Pinakolon mit 300 ml Diethylether und 180 ml Dioxan werden bei 35-40° C unter Rühren 35,00 g Brom getropft. Gegen Ende der Reaktion verfärbt sich die Lösung. Sie wird mit Natriumbisulfit/Wasser bis zum Verschwinden der Bromfarbe geschüttelt, mit Wasser gewaschen und am Rotationsverdampfer eingeengt. Das dabei ausfallende, schwach orangefarbene Produkt wird aus n-Hexan umkristallisiert.

Ausbeute: 206 g (81 % der Theorie) 1,1-Dibrom-3,3-dimethylbutanon-2 vom Schmelzpunkt 75° C.

Beispiel 2

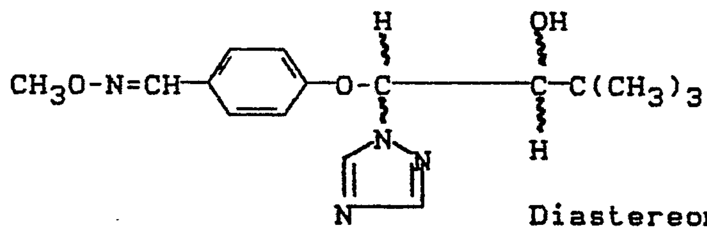

Diastereomeres (A)

Eine Mischung von 0,3 g (0,006 Mol) Kaliumhydroxid-Pulver in 40 ml trockenem n-Butanol wird bei Raumtemperatur unter Rühren mit 1,4 g (0,02 Mol) 1,2,4-Triazol versetzt. Man erhitzt das Reaktionsgemisch auf 65°C und tropft unter Rühren eine Lösung von 5,0 g (0,02 Mol) 3,3-Dimethyl-1-(4-methoximinomethylphenoxy)-trans-1,2-epoxy-butan in 10 ml trockenem n-Butanol hinzu. Das Reaktionsgemisch wird noch 20 Stunden unter Rückfluß nachgerührt und dann durch Abziehen des Lösungsmittels unter vermindertem Druck eingeengt. Man erhält 8 g eines Rohproduktes, das nach GC- und HPLC-Bestimmung zu 1,97 g aus dem Diastereomeren (A) des 3,3-Dimethyl-1-(4-methoximinomethyl-phenoxy)-1-(1,2,4-triazol-1-yl)-butan-2-ols besteht. Die Ausbeute errechnet sich danach zu 31 % der Theorie. Das Diastereomere (B) konnte nicht nachgewiesen werden.

Vergleichsbeispiel

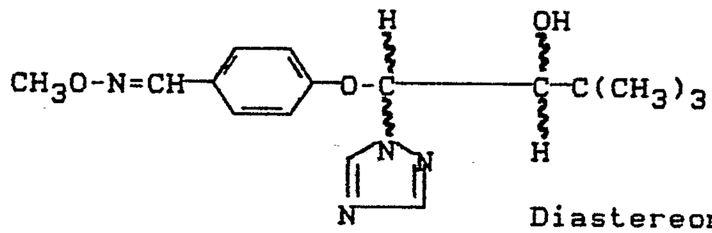

Diastereomeres (B)

Eine Lösung von 50 g (1,3 Mol) Natriumborhydrid in 300 ml Wasser wird unter Rühren in eine Lösung von 316 g (1 Mol) 3,3-Dimethyl-1-(4-methoximinomethyl-phenoxy)-1-(1,2,4-triazol-1-yl)-butan-2-on eingetropft. Man rührt 24 Stunden bei Raumtemperatur, versetzt dann mit weiteren 30 g (0,8 Mol) Natriumborhydrid und rührt noch einmal 24 Stunden bei Raumtemperatur. Zur anschließenden Aufarbeitung wird das Reaktionsgemisch in Wasser gegossen, und das entstehende Gemisch wird mehrfach mit Methylenchlorid extrahiert. Die vereinigten organischen Phasen werden nach dem Trocknen unter vermindertem Druck eingeengt. Man erhält 264 g (83 % der Theorie) an 3,3-Dimethyl-1-(4-methoximinomethyl-phenoxy)-1-(1,2,4-triazol-1-yl)-butan-2-ol (Diastereomeren-Gemisch) in Form eines zähen Öles.

Ein Teil des erhaltenen Produktes wird über eine Kieselgel-Säule mit dem Fließmittel Toluol/Aceton = 8:2 chromatographiert. Nach dem Einengen des Eluates erhält man ein öliges Produkt, das nach kurzem Stehenlassen bei Raumtemperatur kristallisiert. Durch Umkristallisation dieses Produktes aus Di-isopropyl-ether erhält man das Diastereomere (B) des 3,3-Dimethyl-1-(4-methoximinomethyl-phenoxy)-1-(1,2,4-triazol-1-yl)-butan-2-ols als Feststoff mit dem Schmelzpunkt 136-140° C.

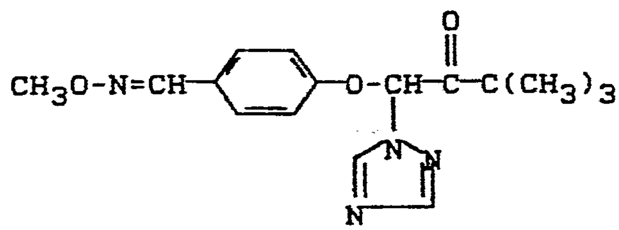

In eine Aufschlämmung von 151 g (1 Mol) 4-Hydroxy-benzaldehyd-O-methyloximether und 150 g (1,1 Mol) Kaliumcarbonat in 800 ml Acetonitril läßt man unter Rühren bei Raumtemperatur eine Lösung von 319,8 g (1,3 Mol) 1-Brom-1-(1,2,4-triazol-1-yl)-3,3-dimethyl-butan-2-on in 100 ml Acetonitril innerhalb von 30 Minuten zutropfen. Nach dem Abklingen der leicht exothermen Reaktion wird 3 Stunden bei 60 bis 65° C gerührt, dann auf Raumtemperatur abgekühlt und in Wasser gegossen. Das entstehende Gemisch wird mehrfach mit Toluol extrahiert. Die vereinigten organischen Phasen werden zweimal mit Wasser gewaschen, dann getrocknet und unter vermindertem Druck eingeengt. Der verbleibende Rückstand wird mit Ligroin versetzt, wobei sich nach dem Anreiben ein kristallines Produkt bildet. Nach dem Trocknen auf Ton erhält man 241 g (76 % der Theorie) an 3,3-Dimethyl-1-(4-methoximinomethyl-phenoxy)-1-(1,2,4-triazol-1-yl)-butan-2-on vom Schmelzpunkt 83 bis 87° C.

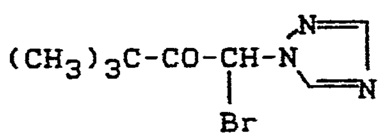

In eine Lösung von 217 g (1,3 Mol) 3,3-Dimethyl-1-(1,2,4-triazol-1-yl)-butan-2-on in 700 ml Eisessig werden unter Rühren bei Raumtemperatur 110 g (1,34 Mol) Natriumacetat

eingetragen, wobei die Temperatur etwas ansteigt. Nach 30-minütigem Nachrühren werden unter leichter Kühlung bei einer Temperatur zwischen 30 und 33°C innerhalb von 30 Minuten 208 g (2,6 Mol) Brom zugetropft. Man läßt 2,5 Stunden bei Raumtemperatur nachrühren, gießt dann in 1.200 ml Wasser und fügt 1.000 ml Methylenchlorid hinzu. Die organische Phase wird zunächst zweimal mit Wasser und dann mit wäßriger Natriumhydrogencarbonat-Lösung gewaschen. Nach dem Trocknen über Natriumsulfat wird unter vermindertem Druck eingeengt. Man erhält 1-Brom-1-(1,2,4-triazol-1-yl)-3,3-dimethyl-butan-2-on in Form eines Rohproduktes, das ohne Reinigung für die weitere Umsetzung verwendet wird.

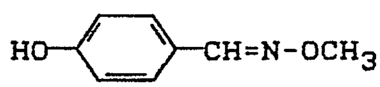

In 1.000 ml Ethanol werden bei Raumtemperatur unter Rühren nacheinander 244 g (2 Mol) 4-Hydroxy-benzaldehyd, 195 g (2,33 Mol) O-Methyl-hydroxylamin-hydrochlorid und 180 g (2,2 Mol) Natriumacetat eingetragen. Danach heizt man innerhalb von 2 Stunden auf Siedetemperatur auf und rührt dann 2 Stunden unter Rückfluß und anschließend noch 16 Stunden bei Raumtemperatur. Zur Aufarbeitung wird das Reaktionsgemisch in 800 ml Wasser gegossen. Das entstehende Gemisch wird zweimal mit je 800 ml Methylenchlorid ausgeschüttelt. Die vereinigten organischen Phasen werden nach dem Trocknen über Natriumsulfat eingeengt. Der Rückstand wird durch Andestillieren unter vermindertem Druck von noch anhaftenden Lösungsmittel-Resten befreit. Man erhält 291 g (96 % der Theorie) an 4-Hydroxy-benzaldehyd-O-methyloximether in Form eines Feststoffes vom Schmelzpunkt 70 bis 72°C.

In den folgenden Verwendungsbeispielen wurden die nachstehend angegebenen Verbindungen bezüglich ihrer fungiziden Eigenschaften geprüft:

(A) = 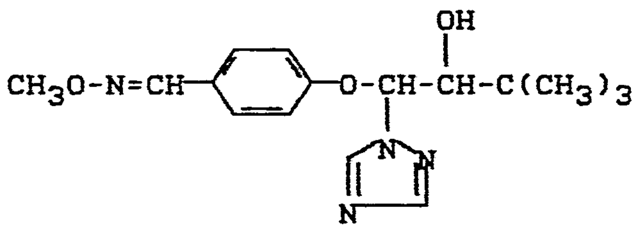

Diastereomerengemisch
(bekannt aus EP-OS 0 076 370)

(B) = 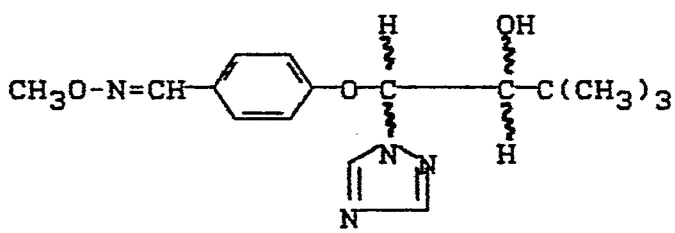

Diastereomeres (B)

(I) = 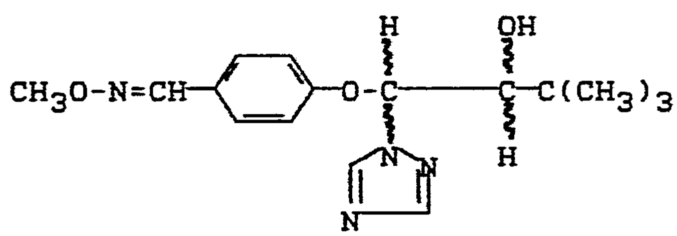

Diastereomeres (A)
(erfindungsgemäß)

Beispiel A

Sphaerotheca-Test (Gurke) / protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

Wirkstoff, Wirkstoffkonzentrationen und Versuchsergebnisse gehen aus der folgenden Tabelle hervor.

Tabelle A

Sphaerotheca-Test (Gurke) / protektiv

| Wirkstoff | Befall in % bei einer Wirkstoffkonzentration von 1 ppm | 0,5 ppm | 0,25 ppm |
|---|---|---|---|
| (A) Diastereomeren-Gemisch | 9 | 15 | 25 |
| (B) Diastereomeres (B) | 28 | 60 | 65 |
| erfindungsgemäß: | | | |
| (I) Diastereomeres (A) | 4 | 10 | 13 |
| - (unbehandelt) | 85 % Befall | | |

Beispiel B

Fusarium culmorum-Test (Weizen) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Weizen sät man mit 2 x 100 Korn 1 cm tief in eine Standarderde und kultiviert ihn im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome.

Wirkstoffe, Wirkstoffkonzentrationen und Versuchsergebnisse gehen aus der folgenden Tabelle hervor:

Tabelle B

Fusarium culmorum-Test (Weizen) / Saatgutbehandlung

| Wirkstoff | Wirkstoffaufwandmenge in mg / kg Saatgut | kranke Pflanzen in % der insgesamt aufgelaufenen Pflanzen |
|---|---|---|
| - (ungebeizt) | - | 81,8 |
| (A) Diastereomeren-Gemisch | 500 | 27,0 |
| erfindungsgemäß: | | |
| (I) Diastereomeres (A) | 500 | 0,0 |

Patentansprüche

1. Diastereomes (A) des 3,3-Dimethyl-1-(4-methoximinomethyl-phenoxy)-1-(1,2,4-triazol-1-yl)-butan-2-ols der Formel

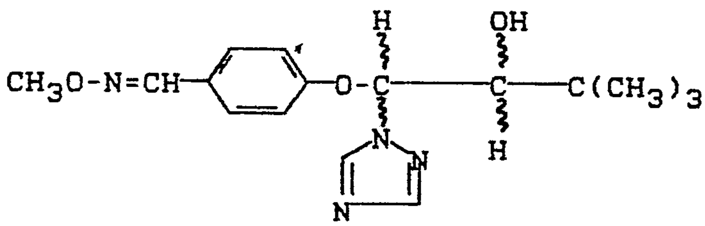

(I)

2. Verfahren zur Herstellung des Diastereomeren (A) des 3,3-Dimethyl-1-(4-methoximinomethyl-phenoxy)-1-(1,2,4-triazol-1-yl)-butan-2-ols der Formel

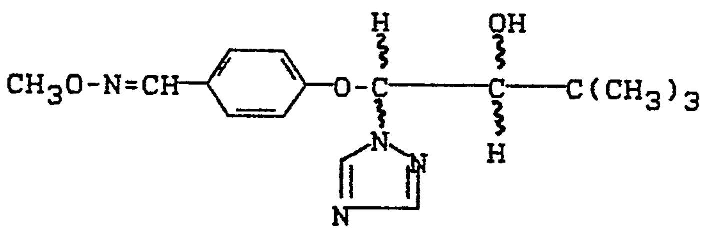

(I)

dadurch gekennzeichnet, daß man 1-Brom-3,3-dimethyl-1,2-epoxy-butan der Formel

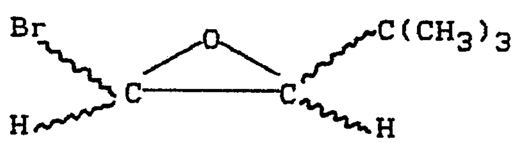

(II)

mit 4-Hydroxy-benzaldehyd-O-methyloximether der Formel

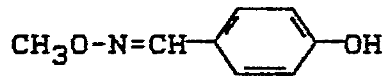 (III)

gegebenenfalls in Gegenwart einer Base sowie gegebenenfalls in Gegenwart eines Verdünnungsmittels umsetzt und das dabei anfallende 3,3-Dimethyl-1-(4-methoximinomethyl-phenoxy)-trans-1,2-epoxy-butan der Formel

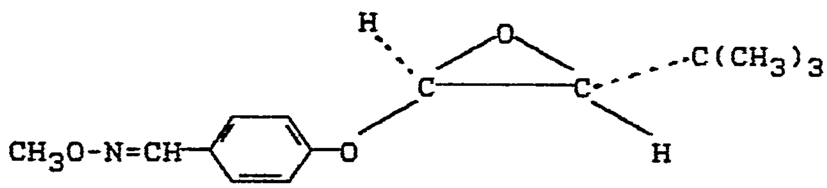 (IV)

in einem zweiten Reaktionsschritt mit 1,2,4-Triazol oder einem Salz des 1,2,4-Triazols gegebenenfalls in Gegenwart einer Base und gegebenenfalls in Gegenwart eines Verdünnungsmittels umsetzt.

3. Fungizide Mittel, dadurch gekennzeichnet durch einen Gehalt an dem Diastereomeren (A) des 3,3-Dimethyl-1-(4-methoximinomethyl-phenoxy)-1-(1,2,4-triazol-1-yl)-butan-2-ols der Formel (I).

4. Verwendung des Diastereomeren (A) des 3,3-Dimethyl-1-(4-methoximinomethyl-phenoxy)-1-(1,2,4-triazol-1-yl)-butan-2-ols der Formel (I) zur Bekämpfung von Pilzen.

5. Verfahren zur Bekämpfung von Pilzen, dadurch gekennzeichnet, daß man das Diastereomere (A) des 3,3-Dimethyl-1-(4-methoximinomethyl-phenoxy)-1-(1,2,4-triazol-1-yl)-butan-2-ols der Formel (I) auf Pilze und/oder deren Lebensraum ausbringt.

6. Verfahren zur Herstellung von fungiziden Mitteln, dadurch gekennzeichnet, daß man das Diastereomere (A) des 3,3-Dimethyl-1-(4-methoximinomethyl-phenoxy)-1-(1,2,4-triazol-1-yl)-butan-2-ols der Formel (I) mit Streckmitteln und/oder oberflächenaktiven Stoffen vermischt.

7. 3,3-Dimethyl-1-(4-methoximino-methyl-phenoxy)-trans-1,2-epoxy-butan der Formel

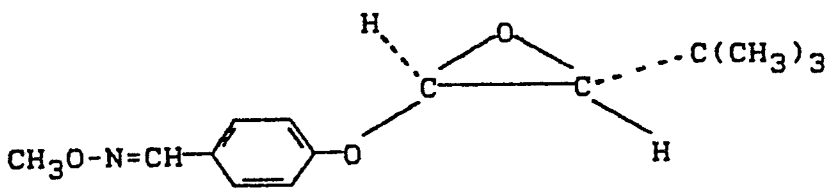

(IV)

0229301

Europäisches Patentamt

# EUROPÄISCHER RECHERCHENBERICHT

Nummer der Anmeldung

EP 86 11 7015

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,D | EP-A-0 076 370 (BAYER AG)<br>* Seite 25, Tabelle, Verbindung 11; Beispiele A,C,E * | 3 | C 07 D 249/08<br>A 01 N 43/653<br>C 07 D 303/48<br>C 07 B 53/00 //<br>C 07 C 131/00 |
| Y | * Seite 25, Tabelle, Verbindung 11; Seite 27, Beispiele A,C-E; Seite 36, Formel IV; Ansprüche 5-7 *<br>--- | 1,2,4-7 | |
| Y | TETRAHEDRON LETTERS, Band 26, Nr. 36, 1985, Seiten 4341-4344, Oxford, GB; J. GASTEIGER et al.: "Diastereospecific synthesis of fungicidal threo- und erythro alpha-hydroxy aminals"<br>* insgesamt *<br>--- | 1,2,4-7 | **RECHERCHIERTE SACHGEBIETE (Int. Cl.4)** |
| Y | EP-A-0 001 414 (BAYER AG)<br>* Seiten 1,2, Seite 3, Zeilen 9-19; Beispiele A-C *<br>--- -/- | 1,2,4-7 | C 07 D 249/00<br>A 01 N 43/00<br>C 07 D 521/00<br>C 07 B 53/00 |

Der vorliegende Recherchenbericht wurde für alle Patentansprüche erstellt.

| Recherchenort | Abschlußdatum der Recherche | Prüfer |
|---|---|---|
| BERLIN | 23-03-1987 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

EP 86 11 7015



## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | TETRAHEDRON LETTERS, Band 26, Nr. 36, 1985, Seiten 4337-4340, Pergamon Press, Oxford, GB; J. GASTEIGER et al.: "Nucleophilic substitution at a saturated carbon atom with retention of configuration: the reaction of trans-2-halo-3-tert-butyloxiranes with phenolates" * insgesamt * | 2 | |
| A | EP-A-0 163 895 (BASF A.G.) * Seite 1, Zeile 8 - Seite 2, Zeile 4 * | 1,3-6 | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| BERLIN | 23-03-1987 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument